# EUROPEAN PATENT APPLICATION

(11) **EP 4 032 881 A1**
(43) Date of publication of application: **27.07.2022**
(21) Application number: 21152776.7
(22) Date of filing: 21.01.2021
(51) Int. Cl.: C07D 307/46, C07H 1/06

(54) **METHOD AND APPARATUS FOR PURIFYING A FURAN DERIVATIVE**

(71) Applicant: Universität Hohenheim, 70599 Stuttgart (DE)
(72) Inventor: Kruse, Andrea, 73249 Wendlingen am Neckar (DE); Swiatek, Katarzyna, 70599 Stuttgart (DE); Olszewski, Maciej, 70599 Stuttgart (DE)
(74) Representative: Altmann Stößel Dick Patentanwälte PartG mbB

(57) **Abstract**

A method for purifying a furan derivative is disclosed. The method comprises providing a reaction mixture (110) comprising a furan derivative and a carbohydrate, wherein the reaction mixture (110) is preferably obtained from a 5-hydroxymethylfurfural synthesis of carbohydrates, wherein the products of the 5-hydroxymethylfurfural synthesis comprise a furan derivative and a carbohydrate, carrying out adsorption of the furan derivative from the reaction mixture (110) at activated carbon (104), carrying out desorption of the furan derivative adsorbed at the activated carbon (104) after a predetermined time by means of a solvent (124) revealing a mixture (134) comprising the solvent (124) and the furan derivative, and separating the solvent (124) from the mixture (134) so as to obtain a purified furan derivative (136). Further, an apparatus (100) for purifying a furan derivative is disclosed.

## Description

### Technical Field

The present invention relates to a method and an apparatus for purifying a furan derivative.

### Background art

The separation of bio-based chemicals is one of the most difficult tasks in the scope of modern bio refinery. The reason for this is a low concentration of organic compounds after a chemical reaction, especially furfurals, 5-hydroxymethylfurfural (5-HMF), and very often thermal instability of those compounds.

So far, the bio-based chemicals, especially furfurals, 5-hydroxymethylfurfural (5-HMF), have been separated by liquid-liquid extraction followed by phase separation and evaporation of the solvent. Currently, some organic solvents immiscible with water are used such as chloroform or methyl isobutyl ketone. Another way to separate the compounds is membrane filtration. Also, a chromatography method for separation of bio-based chemicals is known.

Despite the advantages provided by these devices and methods described above, there are still some drawbacks. Particularly, the disadvantage of liquid-liquid extraction is the usage of toxic organic solvents such as chloroform which often require restrictive regulations regarding handling and disposal. The solvents present in the reaction mixture generate additional pollution. Organic solvents often have a lack of selectivity to the desired compounds. Therefore, byproducts are extracted as well. Further, usually, a large amount of solvent is necessary, which leads to high energy consumption for the evaporation of the solvent and generates enormous operating costs. Membrane filtration has a limited flow capacity, can operate at restricted concentration, and it is challenging to scale up economically. The membrane filters have high investment costs and operating costs related to membrane channel clogging and membrane surface fouling. Chromatography separation is not scalable for industrial application due to injection-like operation. The chromatography column is prone to clogging and requires high operating pressures. Usually, it is regarded as the most expensive method, which could be applied as separation unit operation.

It is therefore desirable to provide a high-efficiency continuous separation of thermally instable, bio-based, especially from aqueous sugar solutions, platform chemicals, especially furfurals, 5-hydroxymethylfurfural (5-HMF), from a reaction mixture after chemical synthesis.

### Summary

This problem is addressed by a method and an apparatus for purifying a furan derivative with the features of the independent claims. Advantageous embodiments which might be realized in an isolated fashion or in any arbitrary combinations are listed in the dependent claims as well as throughout the specification.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, it shall be noted that the terms "at least one", "one or more" or similar expressions indicating that a feature or element may be present once or more than once typically will be used only once when introducing the respective feature or element. In the following, in most cases, when referring to the respective feature or element, the expressions "at least one" or "one or more" will not be repeated, non-withstanding the fact that the respective feature or element may be present once or more than once.

Further, as used in the following, the terms "preferably", "more preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in such way with other optional or non-optional features of the invention.

In a first aspect of the invention, there is provided a method for purifying a furan derivative, comprising the following steps, preferably in the given order:
- providing a reaction mixture comprising a furan derivative and a carbohydrate,
- carrying out adsorption of the furan derivative from the reaction mixture at activated carbon,
- carrying out desorption of the furan derivative adsorbed at the activated carbon after a predetermined time by means of a solvent revealing a mixture comprising the solvent and the furan derivative, and
- separating the solvent from the mixture so as to obtain a purified furan derivative.

The purification process has a simple construction and can operate with a solvent which is rather safe under human health and environmental aspects and which can be bio-based, for example, ethanol, acetone. The adsorption and desorption process can be carried out at low temperature and low pressure such as temperatures below 110°C and atmospheric pressure. Particularly, the degradation of the final product is minimized as the separation at the activated carbon can be carried out at temperature below 110°C, which prevents the thermal decomposition of the products. In a step subsequent to the separation and desorption, the solvent can be removed by evaporation at vacuum allowing to decrease the boiling point of the solvent. The process water after the separation of the desired product at the activated carbon is rich in carbohydrate such as sugars. Therefore, it can be recirculated, mixed with feedstock and feed again into a reactor or used in other ways where a sugar solution is needed. The significant advantage of the invention is that a wide concentration range of desired product in the feed stream can be processed. Another advantage of the method described herein is the removal of sugars from the aqueous reaction mixture before other components are removed. In addition, the process can be fully bio-based. For example, the activated carbon can be produced from agricultural waste and bio-ethanol can be used as solvent which significantly improves life-cycle assessment. Moreover, the process water is not contaminated with harmful solvents.

The term "furan derivative" as used herein is a broad term and is to be given its ordinary and customary meaning to a person of ordinary skill in the art and is not to be limited to a special or customized meaning. The term specifically may refer, without limitation, to a compound containing the furan ring. Thus, the term refers to furan and its furfural derivatives.

The term "purifying" as used herein is a broad term and is to be given its ordinary and customary meaning to a person of ordinary skill in the art and is not to be limited to a special or customized meaning. The term specifically may refer, without limitation, to a process of rendering something pure, i.e. clean of foreign elements and/or pollution.

The term "reaction mixture" as used herein is a broad term and is to be given its ordinary and customary meaning to a person of ordinary skill in the art and is not to be limited to a special or customized meaning. The term specifically may refer, without limitation, to a mixture containing reactants and products of 5-HMF synthesis from carbohydrates. These carbohydrates can be market available sugars, e.g. fructose/glucose, or obtained by hydrolysis of polysaccharide-based biomass, e.g. lignocellulose.

The term "adsorption" as used herein is a broad term and is to be given its ordinary and customary meaning to a person of ordinary skill in the art and is not to be limited to a special or customized meaning. The term specifically may refer, without limitation, to the adhesion of atoms, ions or molecules from a gas, liquid or dissolved solid to a surface. This process creates a film of the adsorbate on the surface of the adsorbent. This process differs from absorption, in which a fluid (the absorbate) is dissolved by or permeates a liquid or solid (the absorbent), respectively. Adsorption is a surface phenomenon, while absorption involves the whole volume of the material, although adsorption does often precede absorption.

The term "activated carbon" as used herein is a broad term and is to be given its ordinary and customary meaning to a person of ordinary skill in the art and is not to be limited to a special or customized meaning. The term specifically may refer, without limitation, to a form of carbon processed to have small, low-volume pores that increase the surface area available for adsorption or chemical reactions. Activated carbon is also called activated charcoal. Activated is sometimes substituted with active. Due to its high degree of microporosity, one gram of activated carbon can have a surface area in excess of 3,000 m² as determined by gas adsorption. An activation level sufficient for useful application may be obtained solely from high surface area. Further chemical treatment often enhances adsorption properties. Activated carbon is usually derived from charcoal. Basically, activated carbon is carbon produced from carbonaceous source materials such as bamboo, coconut husk, willow peat, wood, coir, lignite, coal, and petroleum pitch.

The term "desorption" as used herein is a broad term and is to be given its ordinary and customary meaning to a person of ordinary skill in the art and is not to be limited to a special or customized meaning. The term specifically may refer, without limitation, to a process whereby a substance is released from or through a surface. The process is the opposite of sorption (that is, either adsorption or absorption). This occurs in a system being in the state of sorption equilibrium between bulk phase (fluid, e.g. gas or liquid solution) and an adsorbing surface (solid or boundary separating two fluids). When the concentration (or pressure) of substance in the bulk phase is lowered, some of the sorbed substance changes to the bulk state. In chemistry, especially chromatography, desorption is the ability for a chemical to move with the mobile phase. The more a chemical desorbs, the less likely it will adsorb, thus instead of sticking to the stationary phase, the chemical moves up with the solvent front. In chemical separation processes, stripping is also referred to as desorption as one component of a liquid stream moves by mass transfer into a vapor phase through the liquid-vapor interface. After adsorption, the adsorbed chemical will remain on the substrate nearly indefinitely, provided the temperature remains low. However, as the temperature rises also desorption is increased.

The term "separating" as used herein is a broad term and is to be given its ordinary and customary meaning to a person of ordinary skill in the art and is not to be limited to a special or customized meaning. The term specifically may refer, without limitation, to a method that converts a mixture or solution of chemical substances into two or more distinct product mixtures. At least one of the fractions resulting from the separation is enriched in one or more of the source mixture's constituents. In some cases, a separation may fully divide the mixture into pure constituents. Separations exploit differences in chemical properties or physical properties (such as size, shape, mass, density, or chemical affinity) between the constituents of a mixture. Processes are often classified according to the particular differences they use to achieve separation. If no single difference can be used to accomplish the desired separation, multiple operations can often be combined to achieve the desired end. The purpose of separation may be analytical, can be used as a lie component in the original mixture without any attempt to save the fractions, or maybe preparative, i.e. to "prepare" fractions or samples of the components that can be saved. The separation can be done on a small scale, effectively a laboratory scale for analytical or preparative purposes, or on a large scale, effectively an industrial scale for preparative purposes, or on some intermediate scale.

Preferably, the reaction mixture is obtained from a 5-hydroxymethylfurfural synthesis of carbohydrates, wherein the products of the 5-hydroxymethylfurfural synthesis comprise a furan derivative and a carbohydrate. Thus, the furan derivative may be produced on an organic basis.

The method may further comprise supplying the reaction mixture to at least one column comprising the activated carbon. Thus, the activated carbon may be provided at a compact device which decreases the space necessary for activated carbon. Further, such a column is well established and easily to integrate in existing apparatus and/or to be scaled-up. Thus, providing such a column is a reliable and well established process.

The term "column" as used herein is a broad term and is to be given its ordinary and customary meaning to a person of ordinary skill in the art and is not to be limited to a special or customized meaning. The term specifically may refer, without limitation, to a tube-shaped device on which is fixed a material called the stationary phase. According to the present disclosure, the stationary phase is activated carbon. The column is used for the separation of a mixture. The mixture is dissolved in a fluid called the mobile phase, which carries it through the column, respectively. The different constituents of the mixture have different affinities for the stationary phase. The different molecules stay longer or shorter on the stationary phase, depending on their interactions with its surface sites. So, they travel at different apparent velocities in the mobile fluid, causing them to separate. The separation is based on the differential partitioning between the mobile and the stationary phases. Subtle differences in a compound's partition coefficient result in differential retention on the stationary phase and thus affect the separation. While small-scale columns range from inner diameters of 0.5 cm and withstand pressures of up to 130 MPa, industrial large scale columns reach diameters of up to 2 m and operate at considerable lower pressures (below 1 MPa). While it is favorable to view the packed bed of a column large scale columns are manufactured from steel due to its superior resilience.

The method may further comprise supplying the solvent to the column so as to desorb the furan derivative from the activated carbon. Thus, the adsorbed furan derivative may be eluted with a solvent while other components such as the carbohydrate pass the column.

The carbohydrate may include at least one monosaccharide, particularly fructose or glucose. Thus, the furan derivative may be produced from rather cheap and bio-based organic components.

The adsorption of the furan derivative from the reaction mixture at the activated carbon may be carried out at a temperature in a range of 5.0°C to 60°C and preferably of 10°C to 50°C. Thus, the adsorption takes places at a rather low temperature which avoids a degradation of the desired product and increases the process efficiency.

The desorption of the furan derivative adsorbed at the activated carbon may be carried out when the activated carbon is saturated. Thus, the desorption may be carried out when the activated carbon is no longer capable to adsorb the furan derivative. With other words, the adsorbing capacity of the activated carbon is completely used. Optionally, the bed of the column can be rinsed with water after desorption to remove residual components adhering to the activated carbon such as solvent, sugars and the like. This further improves the quality of the 5-hydroxymethylfurfural at the end.

The solvent may be selected from the group consisting of C1 to C10 alkanol, C3 to C10 alkanone, mixtures of two or more C1 to C10 alkanols, mixtures of two or more C3 to C10 alkanones, and mixtures of one or more C1 to C10 alkanol(s) with one or more C3 to C10 alkanone(s). Thus, a broad range of commercially available solvents may be used.

The term "C1 to C10 alkanol" comprises alkanols with straight alkyl chain having one or more hydroxyl group(s), and alkanols with branched alkyl chain having one or more hydroxyl group(s), wherein each hydroxyl group is independently selected from the group consisting of primary hydroxyl group, secondary hydroxyl group and tertiary hydroxyl group. The term "C3 to C10 alkanone" comprises alkanones having structural formula R₁ -C(=O)-R₂, wherein R₁ and R₂ are independently from each other selected from branched C1 to C9 alkyl groups and straight C1 to C9 alkyl groups, wherein the number of all carbon atoms comprised in R₁ -C(=O)-R₂ is an integer in the range of from 3 to 10.

The solvent may comprise at least ethanol and/or acetone. Thus, solvents, which are rather safe or harmless under human health and environmental aspects, may be used.

The desorption of the furan derivative adsorbed at the activated carbon may be carried out at a temperature at which the solvent is in a liquid state. Basically, it is recommended to perform the desorption at an elevated temperature to improve the efficiency of desorption. However, the desorption medium should stay in the liquid phase. The residence time of the feed strongly depends on the initial concentration of desired products (furfurals, especially 5-HMF) in the reaction mixture. For a higher concentration of the adsorbate in the reaction mixture longer dwell time is recommended.

The desorption of the furan derivative adsorbed at the activated carbon may be carried out at a temperature below a boiling point of the solvent, preferably 2°C to 20°C and more preferably 5°C to 10°C below a boiling point of the solvent. Thus, it is ensured that the solvent is in the liquid state throughout the desorption process.

The activated carbon may preferably be a biological based activated carbon, particularly made of coconut or wood. Thus, bio-based and/or agricultural residues such as straw or nutshells may be used as activated carbon which increases the ecological efficiency. Needless to say, other types of activated carbon than biological based activated carbon may alternatively be used such as coal based active carbon. Particularly, the activated carbon may be produced from carbonaceous source materials such as bamboo, coconut husk, willow peat, wood, coir, lignite, coal, and petroleum pitch.

The solvent may be separated from the mixture revealed from desorption from the activated carbon by means of evaporation. Thus, after desorption, the solvent is evaporated. After the evaporation of the desorption medium, the concentrated and purified product (furfurals, especially 5-HMF) is obtained.

The evaporation may be atmospheric distillation or vacuum evaporation. Thus, the solvent may be evaporated by means of a well-established and cost efficient process.

The method may further comprise recovering the solvent after separating the solvent from the mixture. Thus, the solvent is not lost but can be reused again.

The method may further comprise returning the recovered solvent. Thus, the recovered desorption medium is recycled back to the process.

The method may further comprise separating the carbohydrate from the reaction mixture while carrying out adsorption of the furan derivative from the reaction mixture at the activated carbon. Thus, also the carbohydrate may be used for further processing.

The adsorption of the furan derivative from the reaction mixture at the activated carbon may be carried out at a pressure of 0.5 to 50 bar absolute. Thus, the adsorption takes place at rather low pressure which simplifies the process and makes it safer regarding work protection.

The method may further comprise alternately supplying the reaction mixture to at least two columns each comprising the activated carbon. Thus, the throughput may be significantly increased as there is always at least one column that may be used for the adsorption process. With other words, rather than a batch process, a continuous process is provided.

Alternately supplying the reaction mixture to the two columns each comprising the activated carbon may include switching between the columns when the activated carbon of one of the columns is saturated. Thus, the throughput is increased.

The furan derivative is selected from the group of furfural derivatives, more preferred from the group consisting of 5-chloromethylfurfural, 5-methylfurfural, and 5-hydroxymethylfurfural, wherein the furan derivative preferably comprises at least 5-hydroxymethylfurfural, more preferably is 5-hydroxymethylfurfural. Thus, the separation of bio-based platform chemicals such as furfurals, 5-HMF from the reaction mixture with good selectivity is provided.

In a second aspect of the invention, there is provided an apparatus for purifying a furan derivative, comprising
a reactor, wherein the reactor comprises activated carbon and an inlet for supplying a reaction mixture comprising a furan derivative and a carbohydrate, wherein the activated carbon is configured to adsorb the furan derivative from the reaction mixture,
a solvent reservoir storing a solvent and being in fluid communication with the reactor, wherein the solvent is configured to desorb a furan derivative adsorbed at the activated carbon, and
a separating device configured to separate solvent from a mixture including furan derivative desorbed from the activated carbon and the solvent so as to obtain a purified furan derivative.

The purification apparatus has a simple construction and can operate with a solvent which is rather safe under human health and environmental aspects and which can be bio-based, for example, ethanol, acetone. The adsorption and desorption process can be carried out by means of the apparatus at low temperatures and low pressure such as temperatures below 110°C and atmospheric pressure. The degradation of the final product is minimized as the separation at the activated carbon can be carried out at a temperature below 110°C, which prevents the thermal decomposition of the products. The process water after the separation of the desired product at the activated carbon is rich in carbohydrate such as sugars. Therefore, it can be recirculated, mixed with feedstock and feed again into a reactor or used in other ways where a sugar solution is needed. The significant advantage of the invention is that a wide concentration range of desired product in the feed stream can be processed. Another advantage of the method described herein is the selective removal of sugars from the aqueous reaction mixture. In addition, the process can be fully bio-based. For example, the activated carbon can be produced from agricultural waste and bio-ethanol can be used as solvent which significantly improves life-cycle assessment. Moreover, the process water is not contaminated with harmful solvents.

Preferably, the reaction mixture is obtained from a 5-hydroxymethylfurfural synthesis of carbohydrates, wherein the products of the 5-hydroxymethylfurfural synthesis comprise a furan derivative and a carbohydrate. Thus, the furan derivative may be produced on an organic basis.

The reactor may comprise at least one column comprising the activated carbon. Thus, the activated carbon may be provided at a compact device which decreases the space necessary for activated carbon. Further, such a column is well established and easily to be integrated in existing apparatus and/or to be scaled-up.

The solvent may be selected from the group consisting of C1 to C10 alkanol, C3 to C10 alkanone, mixtures of two or more C1 to C10 alkanols, mixtures of two or more C3 to C10 alkanones, and mixtures of one or more C1 to C10 alkanol(s) with one or more C3 to C10 alkanone(s). Thus, a broad range of commercially available solvents may be used.

The solvent may comprise at least ethanol and/or acetone. Thus, solvents, which are rather safe or harmless under human health and environmental aspects, may be used.

The activated carbon may preferably be a biological based activated carbon, particularly made of coconut or wood. Thus, bio-based and/or agricultural residues may be used as activated carbon which increases the ecological efficiency. Needless to say, other types of activated carbon than biological based activated carbon may alternatively be used such as coal based active carbon. Particularly, the activated carbon may be produced from carbonaceous source materials such as bamboo, coconut husk, willow peat, wood, coir, lignite, coal, and petroleum pitch.

The separating device may comprise an evaporator configured to evaporate the solvent from the mixture. Thus, after desorption, the solvent is evaporated. After the evaporation of the desorption medium, the concentrated and purified product (furfurals, especially 5-HMF) is obtained.

The apparatus may further comprise a recovering device configured to recover the solvent separated from the mixture. Thus, the solvent is not lost but can be reused again.

The reactor may comprise at least two columns each comprising the activated carbon, wherein the reactor may further comprise a switching device configured to switch a supply of the reaction mixture between the two columns. Thus, the throughput is increased.

Further disclosed and proposed herein is a computer program including computer-executable instructions for performing the method according to the present invention in one or more of the embodiments enclosed herein when the program is executed on a computer or computer network. Specifically, the computer program may be stored on a computer-readable data carrier and/or on a computer-readable storage medium.

As used herein, the terms "computer-readable data carrier" and "computer-readable storage medium" specifically may refer to non-transitory data storage means, such as a hardware storage medium having stored thereon computer-executable instructions. The computer-readable data carrier or storage medium specifically may be or may comprise a storage medium such as a random-access memory (RAM) and/or a read-only memory (ROM).

Thus, specifically, one, more than one or even all of method steps a) to d) as indicated above may be performed by using a computer or a computer network, preferably by using a computer program.

Further disclosed and proposed herein is a computer program product having program code means, in order to perform the method according to the present invention in one or more of the embodiments enclosed herein when the program is executed on a computer or computer network. Specifically, the program code means may be stored on a computer-readable data carrier and/or on a computer-readable storage medium.

Further disclosed and proposed herein is a data carrier having a data structure stored thereon, which, after loading into a computer or computer network, such as into a working memory or main memory of the computer or computer network, may execute the method according to one or more of the embodiments disclosed herein.

Further disclosed and proposed herein is a computer program product with program code means stored on a machine-readable carrier, in order to perform the method according to one or more of the embodiments disclosed herein, when the program is executed on a computer or computer network. As used herein, a computer program product refers to the program as a tradable product. The product may generally exist in an arbitrary format, such as in a paper format, or on a computer-readable data carrier and/or on a computer-readable storage medium. Specifically, the computer program product may be distributed over a data network.

Finally, disclosed and proposed herein is a modulated data signal which contains instructions readable by a computer system or computer network, for performing the method according to one or more of the embodiments disclosed herein.

Referring to the computer-implemented aspects of the invention, one or more of the method steps or even all of the method steps of the method according to one or more of the embodiments disclosed herein may be performed by using a computer or computer network. Thus, generally, any of the method steps including provision and/or manipulation of data may be performed by using a computer or computer network. Generally, these method steps may include any of the method steps, typically except for method steps requiring manual work, such as providing the samples and/or certain aspects of performing the actual measurements.

Specifically, further disclosed herein are:
- a computer or computer network comprising at least one processor, wherein the processor is adapted to perform the method according to one of the embodiments described in this description,
- a computer loadable data structure that is adapted to perform the method according to one of the embodiments described in this description while the data structure is being executed on a computer,
- a computer program, wherein the computer program is adapted to perform the method according to one of the embodiments described in this description while the program is being executed on a computer,
- a computer program comprising program means for performing the method according to one of the embodiments described in this description while the computer program is being executed on a computer or on a computer network,
- a computer program comprising program means according to the preceding embodiment, wherein the program means are stored on a storage medium readable to a computer,
- a storage medium, wherein a data structure is stored on the storage medium and wherein the data structure is adapted to perform the method according to one of the embodiments described in this description after having been loaded into a main and/or working storage of a computer or of a computer network, and
- a computer program product having program code means, wherein the program code means can be stored or are stored on a storage medium, for performing the method according to one of the embodiments described in this description, if the program code means are executed on a computer or on a computer network.

Summarizing and without excluding further possible embodiments, the following embodiments may be envisaged:
Embodiment 1: A method for purifying a furan derivative, comprising:
   - providing a reaction mixture comprising a furan derivative and a carbohydrate, wherein the reaction mixture is preferably obtained from a 5-hydroxymethylfurfural synthesis of carbohydrates, wherein the products of the 5-hydroxymethylfurfural synthesis comprise a furan derivative and a carbohydrate,
   - carrying out adsorption of the furan derivative from the reaction mixture at activated carbon,
   - carrying out desorption of the furan derivative adsorbed at the activated carbon after a predetermined time by means of a solvent revealing a mixture comprising the solvent and the furan derivative, and
   - separating the solvent from the mixture so as to obtain a purified furan derivative.
Embodiment 2: The method according to the preceding embodiment, further comprising supplying the reaction mixture to at least one column comprising the activated carbon.
Embodiment 3: The method according to the preceding embodiment, further comprising supplying the solvent to the column so as to desorb the furan derivative from the activated carbon.
Embodiment 4: The method according to any one of the preceding embodiments, wherein the carbohydrate includes at least one monosaccharide, particularly fructose or glucose.
Embodiment 5: The method according to any one of the preceding embodiments, wherein the adsorption of the furan derivative from the reaction mixture at the activated carbon is carried out at a temperature in a range of 5.0°C to 60°C and preferably of 10°C to 50°C.
Embodiment 6: The method according to any one of the preceding embodiments, wherein the desorption of the furan derivative adsorbed at the activated carbon is carried out when the activated carbon is saturated.
Embodiment 7: The method according to any one of the preceding embodiments, wherein the solvent is selected from the group consisting of C1 to C10 alkanol, C3 to C10 alkanone, mixtures of two or more C1 to C10 alkanols, mixtures of two or more C3 to C10 alkanones, and mixtures of one or more C1 to C10 alkanol(s) with one or more C3 to C10 alkanone(s).
Embodiment 8: The method according to the preceding embodiment, wherein the solvent comprises at least ethanol and/or acetone.
Embodiment 9: The method according to any one of the preceding embodiments, wherein the desorption of the furan derivative adsorbed at the activated carbon is carried out at a temperature where the solvent is in a liquid state.
Embodiment 10: The method according to any one of the preceding embodiments, wherein the desorption of the furan derivative adsorbed at the activated carbon is carried out at a temperature below a boiling point of the solvent, preferably 2°C to 20°C and more preferably 5°C to 10°C below a boiling point of the solvent.
Embodiment 11: The method according to any one of the preceding embodiments, wherein the activated carbon is made from a carbonaceous source material and preferably is a biological based activated carbon, particularly made of coconut or wood.
Embodiment 12: The method according to any one of the preceding embodiments, wherein the solvent is separated from the mixture revealed from desorption from the activated carbon by means of evaporation.
Embodiment 13: The method according to the preceding embodiment, wherein the evaporation is atmospheric distillation or vacuum evaporation.
Embodiment 14: The method according to any one of the preceding embodiments, further comprising recovering the solvent after separating the solvent from the mixture.
Embodiment 15: The method according to the preceding embodiment, further comprising returning the recovered solvent.
Embodiment 16: The method according to any one of the preceding embodiments, further comprising separating the carbohydrate from the reaction mixture while carrying out adsorption of the furan derivative from the reaction mixture at the activated carbon.
Embodiment 17: The method according to any one of the preceding embodiments, wherein adsorption of the furan derivative from the reaction mixture at the activated carbon is carried out at a pressure of 0.5 to 50 bar absolute.
Embodiment 18: The method according to any one of the preceding embodiments, further comprising alternately supplying the reaction mixture to at least two columns each comprising the activated carbon.
Embodiment 19: The method according to the preceding embodiment, wherein alternately supplying the reaction mixture to the two columns each comprising the activated carbon includes switching between the columns when the activated carbon of one of the columns is saturated.
Embodiment 20: The method according to any one of the preceding embodiments, wherein the furan derivative is selected from the group of furfural derivatives, more preferred from the group consisting of 5-chloromethylfurfural, 5-methylfurfural, and 5-hydroxymethylfurfural, wherein the furan derivative preferably comprises at least 5-hydroxymethylfurfural, more preferred is 5-hydroxymethylfurfural.
Embodiment 21: An apparatus for purifying a furan derivative, comprising
   a reactor, wherein the reactor comprises activated carbon and an inlet for supplying a reaction mixture comprising a furan derivative and a carbohydrate, wherein the reaction mixture is preferably obtained from a 5-hydroxymethylfurfural synthesis of carbohydrates, wherein the products of the 5-hydroxymethylfurfural synthesis comprise a furan derivative and a carbohydrate, wherein the activated carbon is configured to adsorb the furan derivative from the reaction mixture,
   a solvent reservoir storing a solvent and being in fluid communication with the reactor, wherein the solvent is configured to desorb a furan derivative adsorbed at the activated carbon, and
   a separating device configured to separate solvent from a mixture including furan derivative desorbed from the activated carbon and the solvent so as to obtain a purified furan derivative.
Embodiment 22: The apparatus according to the preceding embodiment, wherein the reactor comprises at least one column comprising the activated carbon.
Embodiment 23: The apparatus according to embodiment 21 or 22, wherein the solvent is selected from the group consisting of C1 to C10 alkanol, C3 to C10 alkanone, mixtures of two or more C1 to C10 alkanol s, mixtures of two or more C3 to C10 alkanones, and mixtures of one or more C1 to C10 alkanol(s) with one or more C3 to C10 alkanone(s).
Embodiment 24: The apparatus according to the preceding embodiment, wherein the solvent comprises at least ethanol and/or acetone.
Embodiment 25: The apparatus according to any one of embodiments 21 to 24, wherein the activated carbon is made from a carbonaceous source material and preferably is a biological based activated carbon, particularly made of coconut or wood.
Embodiment 26: The apparatus according to any one of embodiments 21 to 25, wherein the separating device comprises an evaporator configured to evaporate the solvent from the mixture.
Embodiment 27: The apparatus according to any one of embodiments 21 to 26, further comprising a recovering device configured to recover the solvent separated from the mixture.
Embodiment 28: The apparatus according to any one of embodiments 21 to 27, wherein the reactor comprises at least two columns each comprising the activated carbon, wherein the reactor further comprises a switching device configured to switch a supply of the reaction mixture between the two columns.

### Short description of the Figures

Further optional features and embodiments will be disclosed in more detail in the subsequent description of embodiments, preferably in conjunction with the dependent claims. Therein, the respective optional features may be realized in an isolated fashion as well as in any arbitrary feasible combination, as the skilled person will realize. The scope of the invention is not restricted by the preferred embodiments. The embodiments are schematically depicted in the Figures. Therein, identical reference numbers in these Figures refer to identical or functionally comparable elements.

In the Figures:
- Figure 1: shows a schematically illustration of an an apparatus for purifying a furan derivative;
- Figure 2: shows breakthrough curves of main components of the reaction mixture; and
- Figure 3: shows desorption curves of the main components during desorption with ethanol.

### Detailed description of the embodiments

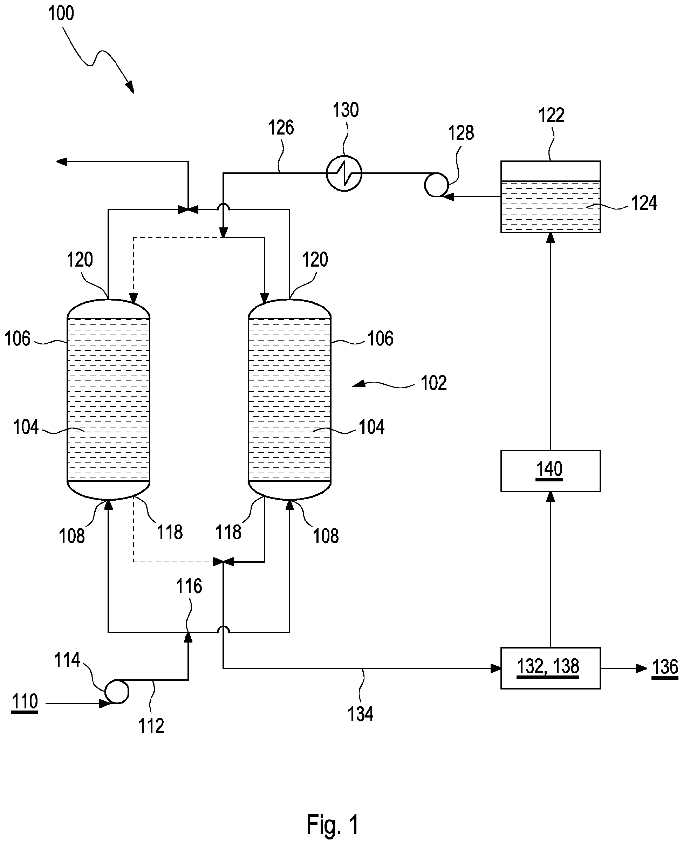

Figure 1 shows a schematically illustration of an apparatus 100 for purifying a furan derivative. The apparatus comprises a reactor 102. The reactor 102 comprises activated carbon 104. Particularly, the reactor 102 comprises at least one column 106 comprising the activated carbon 104. In the present embodiment, the reactor 102 comprises two columns 106. The two columns 106 are arranged in parallel. The activated carbon 104 is made from a carbonaceous source material. Preferably, the activated carbon 104 is a biological based activated carbon. The activated carbon 104 may be made of agricultural residues such as lignocellulose. Particularly, the activated carbon 104 is made of coconut or wood.

The reactor 102 further comprises an inlet 108 for supplying a reaction mixture 110 comprising a furan derivative and a carbohydrate. The reaction mixture 110 may be an aqueous reaction mixture and further includes process water. The furan derivative is selected from the group of furfural derivatives, more preferred from the group consisting of 5-chloromethylfurfural, 5-methylfurfural, and 5-hydroxymethylfurfural. Preferably, the furan derivative comprises at least 5-hydroxymethylfurfural and more preferably is 5-hydroxymethylfurfural. The reaction mixture 110 is preferably obtained from a 5-hydroxymethylfurfural synthesis of carbohydrates. The products of the 5-hydroxymethylfurfural synthesis comprise a furan derivative and a carbohydrate, wherein the activated carbon 104 is configured to adsorb the furan derivative from the reaction mixture 110. Merely as an example, the inlet 108 is located at a bottom end of the columns 106. The inlet 108 may be connected to a reaction mixture supply line 112 including a first pump 114 configured to deliver the reaction mixture 110. The reactor 102 further comprises a switching device 116 configured to switch a supply of the reaction mixture between the two columns 106. The reactor 102 further comprises a first outlet 118. Merely as an example, the first outlet 118 is located at the bottom end of the columns 106. The reactor 102 further comprises a second outlet 120. Merely as an example, the second outlet 120 is located at the top end of the columns 106.

The apparatus 100 further comprises a solvent reservoir 122 storing a solvent 124 and being in fluid communication with the reactor 102. Particularly, the solvent reservoir 122 is in fluid communication with the at least one column 106. In the present embodiment, the solvent reservoir 118 is connected to the reactor 102 by means of a solvent supply line 126. Merely as an example, the solvent supply line 126 is connected to a top end of the columns 106. The solvent supply line 126 includes a second pump 128 configured to deliver the solvent 124 from the solvent reservoir 122 to the columns 106. The solvent supply line 126 further includes a heating device 130 configured to preheat the solvent 124. The solvent is configured to desorb the furan derivative adsorbed at the activated carbon 104. The solvent 124 is selected from the group consisting of C1 to C10 alkanol, C3 to C10 alkanone, mixtures of two or more C1 to C10 alkanols, mixtures of two or more C3 to C10 alkanones, and mixtures of one or more C1 to C10 alkanol(s) with one or more C3 to C10 alkanone(s). Particularly, the solvent 124 comprises at least ethanol and/or acetone.

The apparatus 100 further comprises a separating device 132 configured to separate the solvent 124 from a mixture 134 including furan derivative desorbed from the activated carbon 104 and the solvent 124 so as to obtain a purified furan derivative 136. The separating device 132 comprises an evaporator 138 configured to evaporate the solvent 124 from the mixture 134. The separating device 132 is in fluid communication with the first outlet 118 of the reactor 102. The apparatus 100 further comprises a recovering device 140 configured to recover the solvent 124 separated from the mixture 134. The recovering device 140 is in fluid communication with the solvent reservoir 122. Thus, the recovering device 140 is configured to return the recovered solvent 124 to the solvent reservoir 122.

The apparatus 100 is configured to be used with a method for purifying a furan derivative as will be describes in further detail below.

The method begins with providing a reaction mixture 110 comprising a furan derivative and a carbohydrate. As mentioned before, the reaction mixture 110 is preferably obtained from a 5-hydroxymethylfurfural synthesis of carbohydrates, wherein the products of the 5-hydroxymethylfurfural synthesis comprise a furan derivative and a carbohydrate. The furan derivative is selected from the group of furfural derivatives, more preferred from the group consisting of 5-chloromethylfurfural, 5-methylfurfural, and 5-hydroxymethylfurfural, wherein the furan derivative preferably comprises at least 5-hydroxymethylfurfural, more preferred is 5-hydroxymethylfurfural. The carbohydrate includes at least one monosaccharide, particularly fructose or glucose. Subsequently, adsorption of the furan derivative from the reaction mixture 110 at the activated carbon 104 is carried out. For this purpose, the reaction mixture 110 is supplied to at least one column 106 comprising the activated carbon 104. As mentioned before, the activated carbon 104 is a biological based activated carbon, particularly made of coconut or wood. The adsorption of the furan derivative from the reaction mixture 100 at the activated carbon is carried out at a temperature in a range of 5.0°C to 60°C and preferably of 10°C to 50°C, such as 25°C. Further, the adsorption of the furan derivative from the reaction mixture 110 at the activated carbon 104 is carried out at a pressure of 0.5 to 50 bar absolute, such as. 1.0 bar absolute. While the adsorption of the furan derivative from the reaction mixture 110 at the activated carbon 104 is carried out, the carbohydrate from the reaction mixture 110 is separated. The furan derivative is efficiently captured on the activated carbon 104 while other compounds such as the carbohydrate and process water are passing through the column 106. The carbohydrate together with the process water may be discharged from the reactor 102 and the column 106, respectively, at the second outlet 120. Optionally, the column 106 and the activated carbon 104 may be rinsed with water to remove impurities.

Subsequently, the method proceeds further with carrying out desorption of the furan derivative adsorbed at the activated carbon 104 after a predetermined time by means of a solvent 124 which reveals a mixture 134 comprising the solvent 124 and the furan derivative. Particularly, the solvent 124 is supplied to the column 106 so as to desorb the furan derivative from the activated carbon 104. For this purpose, the solvent 124 is supplied from the solvent reservoir 122 to the column 106 by means of the second pump 128 and enters the column 106 through the solvent supply line 126. As described before, the solvent 124 is selected from the group consisting of C1 to C10 alkanol, C3 to C10 alkanone, mixtures of two or more C1 to C10 alkanols, mixtures of two or more C3 to C10 alkanones, and mixtures of one or more C1 to C10 alkanol(s) with one or more C3 to C10 alkanone(s). In the present embodiment, the solvent 124 comprises at least ethanol and/or acetone. The desorption of the furan derivative adsorbed at the activated carbon 104 is carried out at a temperature where the solvent 124 is in a liquid state. More particularly, the desorption of the furan derivative adsorbed at the activated carbon 124 is carried out at a temperature below a boiling point of the solvent 124, preferably 2°C to 20°C and more preferably 5°C to 10°C below a boiling point of the solvent 124 such as 5°C below a boiling point of the solvent 124. The residence time of the feed strongly depends on the initial concentration of desired products furfurals, especially 5-HMF, in the reaction mixture 110. For a higher concentration of the adsorbate in the reaction mixture 110 longer dwell time is recommended. The point of time when the desorption process is initiated may depend on a load of the activated carbon 104. Particularly, the desorption of the furan derivative adsorbed at the activated carbon 104 is carried out when the activated carbon 104 is saturated. Optionally, the reaction mixture 110 is alternately supplied to at least two columns 106 each comprising the activated carbon 104 as shown in Figure 1. This step may include switching between the columns 106 when the activated carbon 104 of one of the columns 106 is saturated. With other words, the reaction mixture 110 is supplied to one of the columns 106 until the activated carbon 104 thereof is saturated. If the activated carbon of the one column 106 is saturated, the reaction mixture 110 is supplied to the other column 106 while the furan derivative of the one column 106 is desorbed. This switching increases the throughput of the reactor 102.

Subsequently, the mixture 134 is discharged from the column 106 and is supplied to the separating device 132. The solvent 124 is separated from the mixture 134 so as to obtain a purified furan derivative 136. The solvent 124 is separated from the mixture 134 revealed from desorption from the activated carbon 104 by means of evaporation at the evaporator 138 of the separating device 132. The evaporation is atmospheric distillation or vacuum evaporation. After the evaporation of the solvent 124, the concentrated and purified product purified furan derivative is obtained. After separating the solvent 124 from the mixture 134, the solvent 124 may be recovered at the recovering device 140. The recovering device 140 returns the recovered solvent 124. Particularly, the recovering device 140 returns the recovered solvent 124 to the solvent reservoir 122.

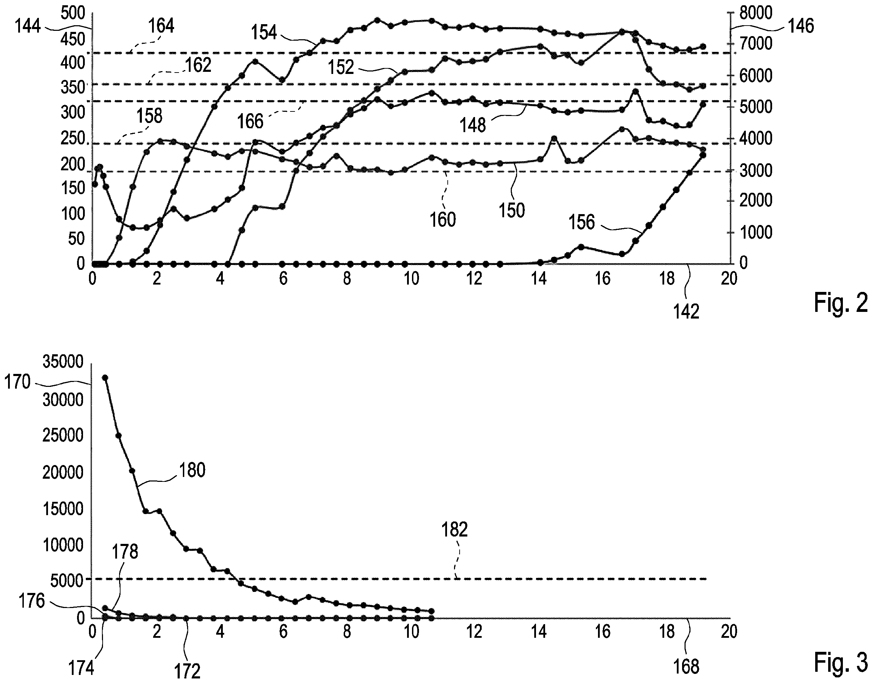

Hereinafter, an example of the method according to the present invention will be described below in further detail with reference to Figures 2 and 3.

The reaction mixture 100 used for the adsorption at the column 106 was process water from a 5-HMF synthesis. For this purpose, 2%wt. fructose solution at pH=2 was passed through a plug-flow reactor at 200 °C and a pressure of 24 bar with residence time of 18 min. The product was collected in tanks and contained about 0.5-1% 5-HMF, unreacted sugar and by products like formic, acetic, and levulinic acid, and also small amounts of furfural.

Then the process water was pumped to the adsorption column 106 filled with activated carbon 104 made of coconut. The residence time of liquid in the adsorption column 106 was 20 min. The samples were collected every 100 ml of outlet solution. The adsorption took place at room temperature, i.e. 20 °C, and atmospheric pressure. After saturation, the bed of the column 106 was rinsed with 200 ml hot demineralised water having a temperature of 75±2 °C. The column 106 was emptied of liquid.

For desorption 99.5% ethanol was used. It was heated up to 65±2 °C at atmospheric pressure. The residence time of ethanol was 20 min. The samples were collected every 100 ml. After desorption, the bed of the column 106 was rinsed with water having room temperature to get rid off the ethanol leftovers.

The thus obtained samples were measured with HPLC. The eluent used was a 0.004 mol L⁻¹ sulphuric acid solution at a flow rate of 0.65 mL min⁻¹. The separation in the column 106 was carried out at a temperature of 25 °C. An Aminex HPX-87H (Biorad, Hercules, CA, USA) separation column was used. Detection took place with RI detector. The bed volume was around 100 cm³. Each collected sample of 100 ml corresponds to 1 bed volume. The resented graphs in Figures 2 and 3 show the relation between concentration after passing through the whole adsorption bed.

Figure 2 shows breakthrough curves of main components of the reaction mixture 110. In Figure 2, the x-axis 142 indicates a bed volume ration which is dimensionless or a percentage value. On the left y-axis 144, a concentration of glucose, formic acid, and levulic acid is given in mg/L. On the right y-axis 146, a concentration of fructose and 5-hydroxymethylfurfural is given in mg/L. Line 148 indicates the concentration graph of glucose, line 150 indicates the concentration graph of formic acid, line 152 indicates the concentration graph of levulic acid, line 154 indicates the concentration graph of fructose and line 156 indicates the concentration graph of 5-hydroxymethylfurfural. Horizontal dashed line 158 is inlet concentration of glucose, horizontal dashed line 160 indicates the inlet concentration formic acid, horizontal dashed line 162 indicates the inlet concentration of levulic acid, horizontal dashed line 164 indicates the inlet concentration of fructose and horizontal dashed line 166 indicates the inlet concentration of 5-hydroxymethylfurfural. As can be seen in Figure 2, 5-hydroxymethylfurfural in the outlet solution or revealed mixture 134 appears much later than the other components. 5-hydroxymethylfurfural is efficiently captured on the activated carbon 104 of the column 106 while the other components such as sugars are passing through the column 106.

Figure 3 shows desorption curves of the main components during desorption with ethanol. In Figure 3, the x-axis 168 indicates a bed volume ration which is dimensionless or a percentage value. The y-axis 170 indicates a concentration of glucose, formic acid, and levulinic acid, fructose and 5-hydroxymethylfurfural in mg/L. Line 172 indicates the concentration graph of glucose, line 174 indicates the concentration graph of formic acid, line 176 indicates the concentration graph of levulinic acid, line 178 indicates the concentration graph of fructose and line 180 indicates the concentration graph of 5-hydroxymethylfurfural. Horizontal dashed line 182 is inlet concentration of 5-hydroxymethylfurfural. As described above, the desorption process is carried out using a flow of short-chained hydrocarbons as solvent 124 such as alcohols or ketones, for example, ethanol or acetone. The course of 5-hydroxymethylfurfural and other components desorption by means of ethanol is exemplarily illustrated in Figure 3. As can be seen above, mainly 5-hydroxymethylfurfural is desorbed. Other components, like sugars or organic acids, are desorbed only in small amounts.

### List of reference numbers

- 100: apparatus
- 102: reactor
- 104: activated carbon
- 106: column
- 108: inlet
- 110: reaction mixture
- 112: reaction mixture supply line
- 114: first pump
- 116: switching device
- 118: first outlet
- 120: second outlet
- 122: solvent reservoir
- 124: solvent
- 126: solvent supply line
- 128: second pump
- 130: heating device
- 132: separating device
- 134: mixture
- 136: purified furan derivative
- 138: evaporator
- 140: recovering device
- 142: x-axis
- 144: left y-axis
- 146: right y-axis
- 148: concentration graph of glucose
- 150: concentration graph of formic acid
- 152: concentration graph of levulic acid
- 154: concentration graph of fructose
- 156: concentration graph of 5-hydroxymethylfurfural
- 158: inlet concentration of glucose
- 160: inlet concentration formic acid
- 162: inlet concentration of levulic acid
- 164: inlet concentration of fructose
- 166: inlet concentration of 5-hydroxymethylfurfural
- 168: x-axis
- 170: y-axis
- 172: concentration graph of glucose
- 174: concentration graph of formic acid
- 176: concentration graph of levulinic acid
- 178: concentration graph of fructose
- 180: concentration graph of 5-hydroxymethylfurfural
- 182: inlet concentration of 5-hydroxymethylfurfural

## Claims

1. A method for purifying a furan derivative, comprising:
- providing a reaction mixture (110) comprising a furan derivative and a carbohydrate, wherein the reaction mixture (110) is preferably obtained from a 5-hydroxymethylfurfural synthesis of carbohydrates, wherein the products of the 5-hydroxymethylfurfural synthesis comprise a furan derivative and a carbohydrate,
- carrying out adsorption of the furan derivative from the reaction mixture (110) at activated carbon (104),
- carrying out desorption of the furan derivative adsorbed at the activated carbon (104) after a predetermined time by means of a solvent (124) revealing a mixture (134) comprising the solvent (124) and the furan derivative, and
- separating the solvent (124) from the mixture (134) so as to obtain a purified furan derivative (136).

2. The method according to the preceding claim, further comprising supplying the reaction mixture (110) to at least one column (106) comprising the activated carbon (104).

3. The method according to the preceding claim, further comprising supplying the solvent (124) to the column (106) so as to desorb the furan derivative from the activated carbon (104).

4. The method according to any one of the preceding claims, wherein the adsorption of the furan derivative from the reaction mixture (110) at the activated carbon (104) is carried out at a temperature in a range of 5.0°C to 60°C and preferably of 10°C to 50°C.

5. The method according to any one of the preceding claims, wherein the desorption of the furan derivative adsorbed at the activated carbon (104) is carried out when the activated carbon (104) is saturated.

6. The method according to any one of the preceding claims, wherein the solvent (124) is selected from the group consisting of C1 to C10 alkanol, C3 to C10 alkanone, mixtures of two or more C1 to C10 alkanols, mixtures of two or more C3 to C10 alkanones, and mixtures of one or more C1 to C10 alkanol(s) with one or more C3 to C10 alkanone(s), wherein the solvent (124) preferably comprises at least ethanol and/or acetone.

7. The method according to any one of the preceding claims, wherein the desorption of the furan derivative adsorbed at the activated carbon (104) is carried out at a temperature where the solvent (124) is in a liquid state, wherein the desorption of the furan derivative adsorbed at the activated carbon (104) is preferably carried out at a temperature below a boiling point of the solvent (124), preferably 2°C to 20°C and more preferably 5°C to 10°C below a boiling point of the solvent (124).

8. The method according to any one of the preceding claims, wherein the activated carbon (104) is made from a carbonaceous source material and preferably is a biological based activated carbon (104), particularly made of coconut or wood.

9. The method according to any one of the preceding claims, wherein the solvent (124) is separated from the mixture (134) revealed from desorption from the activated carbon (104) by means of evaporation, wherein the evaporation is preferably atmospheric distillation or vacuum evaporation.

10. The method according to any one of the preceding claims, further comprising recovering the solvent (124) after separating the solvent (124) from the mixture (134).

11. The method according to any one of the preceding claims, further comprising separating the carbohydrate from the reaction mixture (110) while carrying out adsorption of the furan derivative from the reaction mixture (110) at the activated carbon (104).

12. The method according to any one of the preceding claims, wherein adsorption of the furan derivative from the reaction mixture (110) at the activated carbon (104) is carried out at a pressure of 0.5 to 50 bar absolute.

13. The method according to any one of the preceding claims, further comprising alternately supplying the reaction mixture (110) to at least two columns (106) each comprising the activated carbon (104), wherein alternately supplying the reaction mixture (110) to the two columns (106) each comprising the activated carbon (104) preferably includes switching between the columns (106) when the activated carbon (104) of one of the columns (106) is saturated.

14. The method according to any one of the preceding claims, wherein the furan derivative is selected from the group of furfural derivatives, more preferred from the group consisting of 5-chloromethylfurfural, 5-methylfurfural, and 5-hydroxymethylfurfural, wherein the furan derivative preferably comprises at least 5-hydroxymethylfurfural, more preferred is 5-hydroxymethylfurfural.

15. An apparatus (100) for purifying a furan derivative, comprising
a reactor (102), wherein the reactor (102) comprises activated carbon (104) and an inlet (108) for supplying a reaction mixture (110) comprising a furan derivative and a carbohydrate, wherein the reaction mixture (110) is preferably obtained from a 5-hydroxymethylfurfural synthesis of carbohydrates, wherein the products of the 5-hydroxymethylfurfural synthesis comprise a furan derivative and a carbohydrate, wherein the activated carbon (104) is configured to adsorb the furfural based compound from the reaction mixture (110),
a solvent reservoir (122) storing a solvent (124) and being in fluid communication with the reactor (102), wherein the solvent (124) is configured to desorb a furan derivative adsorbed at the activated carbon (104), and
a separating device (132) configured to separate solvent (124) from a mixture (134) including furan derivative desorbed from the activated carbon (104) and the solvent (124) so as to obtain a purified furan derivative (136).
